(19) **Europäisches Patentamt**
**European Patent Office**
**Office européen des brevets**

(11) **EP 4 670 633 A1**

(12) **EUROPEAN PATENT APPLICATION**

(43) Date of publication:
**31.12.2025 Bulletin 2026/01**

(21) Application number: **24184436.4**

(22) Date of filing: **25.06.2024**

(51) International Patent Classification (IPC):
*A61B 5/349* (2021.01)     *A61B 5/00* (2006.01)
*A61B 5/366* (2021.01)     *G06N 3/044* (2023.01)
*G06N 3/0464* (2023.01)   *G16H 50/20* (2018.01)

(52) Cooperative Patent Classification (CPC):
**A61B 5/349; A61B 5/366; A61B 5/7221;**
**A61B 5/7267; G06N 3/045; G06N 3/08;**
A61B 5/0006; G06N 3/044; G06N 3/0464;
G16H 50/20

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventor: **BONOMI, Alberto Giovann**
**Eindhoven (NL)**

(74) Representative: **Philips Intellectual Property &**
**Standards**
**High Tech Campus 52**
**5656 AG Eindhoven (NL)**

(54) **ELECTROCARDIOGRAPHIC CARDIAC MONITORING**

(57)     Proposed concepts thus aim to provide schemes, solutions, concepts, designs, methods, and systems pertaining to determining the suitability of electrocardiogram signals for use in monitoring a cardiac system of a subject. In particular, embodiments aim to provide a method for automatically assessing the suitability of each of a plurality of electrocardiogram signals based on signal-specific activation patterns of a neural network model configured to take the plurality of ultrasound images as inputs and output a property of the plurality of ECG signals.

FIG. 1

**Description**

FIELD OF THE INVENTION

The invention relates to the field of cardiac monitoring, and more specifically to the field of electrocardiogram monitoring of cardiac systems.

BACKGROUND OF THE INVENTION

Electrocardiograms (ECGs) are routinely used to monitor cardiac electrical activity. During an ECG, electrode sensors are placed on the skin to measure small scale electrical signals that originate from the heart. ECG measurements are incorporated in numerous care pathways and are beneficial for the diagnosis of cardiac diseases and the identification of cardiac anomalies. In clinical practice, multiple ECG signals may be obtained during a single ECG examination. Typical ECG monitors involve multiple ECG channels (or leads) which are each associated with a different ECG sensor placed on the patient's skin and which each have their own associated ECG signal.

There are many ways in which ECG signals may be used for patient monitoring, and these different methods may have different ECG channel configuration requirements. Cardiac diagnosis typically requires 12-lead ECG recordings, or 1-3 ECG leads Holter monitoring. In critical care and telemetry wards patients are typically monitored using 4 ECG lead systems for real-time arrythmia detection and patient surveillance. Some of these functionalities are achieved via automatic interpretation of the ECG signals.

Noise is often present in ECG signals as the ECG comprises small scale electrical impulses that may be easily affected by patient movement or by motion of the ECG wires. Different leads of an ECG monitor may suffer different levels of noise and therefore the different ECG signals obtained during an ECG exam may vary in their quality.

There is often a redundancy in the information carried by the signal from different ECG channels obtained during an ECG examination. The level of noise present in the different ECG signals is difficult to distinguish from the real cardiac signal. It may therefore be difficult to select which of the plurality of ECG signals obtained during an ECG examination to use in further processing steps to achieve the highest reliability and accuracy in any interpretation or diagnosis that is determined as a result.

SUMMARY OF THE INVENTION

The invention is defined by the claims.

According to examples in accordance with an aspect of the invention, there is provided a method for determining the suitability of electrocardiogram (ECG) signals for monitoring the cardiac system of a subject.

The method comprises: obtaining a plurality of ECG signals for monitoring the cardiac system of the subject; processing the plurality of ECG signals with a neural network model to determine a property of the plurality of ECG signals; for each ECG signal of the plurality of ECG signals determining an activation pattern associated with the ECG signal, the activation pattern describing a signal-specific activation level of layers of the neural network model when the neural network model is used to determine the property of the plurality of ECG signals; and for each ECG signal of the plurality of ECG signals determining a quality value of the ECG signal, based on the determined activation patterns, indicating the suitability of the ECG signal for monitoring the cardiac system of the subject.

Proposed concepts thus aim to provide schemes, solutions, concepts, designs, methods, and systems pertaining to determining the suitability of electrocardiogram signals for monitoring the cardiac system of a subject. In particular, embodiments aim to provide a method for automatically assessing the suitability of each of a plurality of electrocardiogram signals based on signal-specific activation patterns of a neural network model configured to take the plurality of ultrasound images as inputs and output a property of the plurality of ECG signals.

ECG monitoring involves the acquisition of multiple ECG signals of the patient which may describe and capture the cardiac activity. Noise is often present in ECG measurements as the ECG is a small amplitude signal describing the electrical activity of the heart as measured on the surface of the skin. Movement of the patient trunk and limbs like arms or legs, the motion of cables or the sensor result in artefacts in the ECG signal that are often hard to remove. Noise present in ECG signals can greatly affect the result of diagnosis and assessment based on the ECG signal - especially if this is achieved using automatic algorithms. Noise can affect different ECG signals (e.g., signals from different leads or channels of an ECG monitor) captured from different parts of the body by differing amounts. It is not always obvious which signals are the least impacted by noise and therefore the most suitable for diagnosis and assessment of the cardiac system.

In neural network models, data provided to an input layer is passed through layers of nodes in the neural network model. At each node weights, a bias, and activation functions are applied to the inputs and the result is passed to nodes in the next layer in the network. An activation map of a neural network model describes the relative importance of each element of the input to the neural network in generating the output. Due to different weights and activation functions applied at each node certain elements of the input data will be more important for the determination of the output and therefore be associated with a higher activation level (where the activation level of a node refers to its output level). The activation pattern associated with an ECG signal of the present invention therefore comprises information about the relative importance of the ECG signal for determining the property of the plurality of ECG signals.

It has been realised that the relative importance of each of a plurality of ECG signals of a cardiac system for determining a common property of the signals may act as a metric for the usefulness of the ECG signal for further processing in diagnosis and monitoring of the cardiac system. The proposed method therefore leverages an activation pattern for each ECG signal describing the activation level of the layers of the neural network for the specific ECG signal, to determine a suitability of the ECG signal for use in monitoring the cardiac system of the subject. This suitability may be used for informed selection of ECG signals for diagnosis and monitoring activities such as arrythmia detection and signal visualization.

In some embodiments, the method may further comprise determining a monitoring requirement value describing a configuration of ECG signals required for monitoring the cardiac system of the subject, and wherein determining for each ECG signal of the plurality of ECG signals, a suitability of the ECG signal for monitoring the cardiac system of the subject is further based on the determined monitoring requirement value. Thus, the suitability assessment of the present invention may further take into account requirements on the configuration of ECG signals required for monitoring the cardiac system as this may be different depending on the presence of any further processing steps intending to be carried out on the data.

In some embodiments, the monitoring requirement value may comprise at least one of: a number of ECG signals required for monitoring the cardiac system of the subject; and a type of ECG signal required for monitoring the cardiac system of the subject. The number and type of ECG signals required for automatic interpretation algorithms may vary significantly and so a quality score that incorporates these features may be particularly beneficial.

In some embodiments, determining the activation pattern associated with an ECG signal of the plurality of ECG signals may comprise: determining a weight value and an activation value associated with each of a plurality of nodes of the neural network when it is used to determine the characteristic of the plurality of ECG signals; and determining the activation pattern for the ECG signal based on the determined weight and activation values of the plurality of nodes of the neural network. This is an efficient way of calculating the activation pattern associated with each of the plurality of ECG signals.

In some embodiments, determining the activation pattern associated with an ECG signal of the plurality of ECG signals may comprise: determining a plurality of feature maps associated with a final layer of the neural network model, calculating a weighted average of the plurality of feature maps; and determining the activation pattern for the ECG signal of the plurality of ECG signals based on a weighted average of the plurality of feature maps. Feature maps may be an accurate and reliable way of determining an activation pattern for each of the ECG signals.

In some embodiments, the property of the plurality of ECG signals may comprise at least one of: a location of heart beats within the plurality of ECG signals; a location of P waves within the plurality of ECG signals; a location of QRS waves within the plurality of ECG signals; a location of T waves within the plurality of ECG signals; a type of beat generating the QRS wave; an interval of a PR segment; an interval of a QT segment; and an interval of an ST segment. Each of these properties are salient characteristics of the ECG signal and therefore a signal-specific activation pattern of a neural network configured to determine any of these properties acts as a reliable metric of the suitability of the ECG signal for monitoring the cardiac parameter of the subject.

In some embodiments, each of the plurality of ECG signals may be obtained by a different lead of an ECG monitor. Therefore, the method allows for the comparison of the suitability of different lead signals from an ECG monitor which all describe the same cardiac signal.

In some embodiments, the quality value of an ECG signal of the plurality of ECG signals comprises a rank of the ECG signal describing a relative importance of the ECG signal compared to the other ECG signals of the plurality of ECG signals for the determination of the property of the plurality of ECG signals. This may allow for the selection of the most suitable combination of ECG signals for diagnosis and further processing.

In some embodiments, the quality value of an ECG signal of the plurality of ECG signals describes the suitability of the ECG signal for monitoring the cardiac system of the subject during a predetermined time period. Noise levels may vary with time and therefore the suitability of the ECG signal may be reassessed for different time periods to allow more accurate selection of the most suitable ECG signals.

In some embodiments, the quality value of the plurality of ECG signals may describe the suitability of the ECG signal for detecting arrythmia of the cardiac system of the subject. Arrythmia detection may be particularly affected by noise and therefore the proposed method may be particularly beneficial for this application.

According to another aspect of the present invention there is provided a computer program comprising computer code means adapted, when said computer program is run on a computer, to implement any of the above described methods.

According to yet another aspect of the present invention there is provided a system for determining the suitability of electrocardiogram signals for use in monitoring a cardiac system of a subject, the system comprising: a data acquisition module configured to obtain a plurality of ECG signals for monitoring the cardiac system of the subject; a data processing module configured to: process the plurality of ECG signals with a neural network model to determine a property of the plurality of ECG signals; and for each ECG signal of the plurality of ECG signals determine an activation pattern associated with the ECG signal the activation pattern describing the signal-specific activation level of layers of the neural network model when the neural network model is used to determine the property of the plurality of ECG signals; and a signal selection module configured to, for each ECG signal of the plurality of ECG signals, determine based on the determined activation patterns, a quality value of the ECG signal indicating the suitability of the ECG signal for monitoring the cardiac system of the subject.

Embodiments may be employed in combination with conventional/existing cardiac monitoring methods and systems. In this way, embodiments may integrate into legacy systems to improve and/or extend their functionality and capabilities. An improved ECG cardiac monitoring system that automatically determines the suitability of ECG signals in monitoring the cardiac system of a subject may therefore be provided by proposed embodiments.

Thus, there may be proposed concepts for determining the suitability of ECG signals for monitoring a cardiac system using activation patterns of a neural network model configured to determine a property of a plurality of ECG signals. These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 is a simplified flow diagram of a method for determining the suitability of ECG signals for monitoring the cardiac system of a subject according to a proposed embodiment;
Fig. 2 is a simplified flow diagram of a method for determining an activation pattern associated with an ECG signal according to a proposed embodiment;
Fig. 3 depicts example activation patterns for two different ECG signals;
Fig. 4 is a simplified block diagram of a system for determining the suitability of ECG signals for monitoring the cardiac system of a subject according to an aspect of the present invention; and
Fig. 5 illustrates an example of a computer within which one or more parts of an embodiment may be employed.

DETAILED DESCRIPTION OF THE EMBODIMENTS

The invention will be described with reference to the Figures.

It should be understood that the detailed description and specific examples, while indicating exemplary embodiments of the apparatus, systems, and methods, are intended for purposed of illustration only and are not intended to limit the scope of the invention. These and other features, aspects, and advantages of the apparatus, systems, and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Implementations in accordance with the present disclosure relate to various techniques, methods, schemes, and/or solutions pertaining to determining the suitability of ECG signals for monitoring the cardiac system of a subject. According to proposed concepts, a number of possible solutions may be implemented separately or jointly. That is, although these possible solutions may be described below separately, two or more of these possible solutions may be implemented in one combination or another.

Embodiments of the invention aim to provide a method for determining the suitability of ECG signals for cardiac monitoring. This is achieved by processing a plurality of ECG signals, intended to be used for monitoring the cardiac system, with a neural network model which is configured to extract some salient global property of the plurality of ECG signals. The activation level of the layers of the neural network when it is used in this way is then analysed to determine a signal-specific activation pattern of the neural network for each of the plurality of ECG signals. This acts as a metric for a predicted suitability of the ECG signal for monitoring the cardiac system.

Proposed concepts thus aim to provide schemes, solutions, concepts, designs, methods, and systems pertaining to automatically determining the suitability of a plurality of ECG signals for use in monitoring a cardiac system based on activation patterns of a neural network model.

During ECG monitoring of the electrical activity of cardiac systems it is common for multiple ECG signals to be obtained simultaneously. Typically, an ECG monitor comprises a plurality of ECG leads (or channels) each connected to a different electrode sensor that is placed somewhere on the surface of the patient's skin. ECG signals measured by such monitors are interpreted by clinical experts for diagnosis and assessment purposes. This process can often be laborious, especially when the ECG is used for a long monitoring period. Machine learning and other automatic interpretation algorithms are often used to streamline the review of the ECG and provide automatic identification of cardiac events. The accuracy of such interpretation algorithms greatly depends on the quality of the ECG waveforms.

There are many sources of noise that may impact ECG signals. The ECG sensor measures the heart electrical activity as perceived on the surface of the patient's skin, the signals are often therefore of very low amplitude and easily affected by noise. Any movement of the patient's limbs or chest, cables, skin-electrode motion and muscle activity may cause artifacts in the ECG signal that are very challenging to remove as their characteristics typically overlaps with those of typical ECG characteristics like QRS, P and T-waves.

ECG waveforms affected by noise are difficult to interpret by automatic algorithms which for example may result in false alarms for cardiac arrhythmia. Rule-based models and feature-based algorithms are examples of automatic analysis that benefits greatly from noise-free signals. These types of algorithms are advantageous as they require low computational resources and are therefore suitable for software embedded products. Hospital patient monitors, telemetry devices and mobile cardiac outpatient telemetry (MCOT) still require relatively simple and computationally lean algorithms for ECG interpretation. The proposed invention may therefore be of great benefit in allowing for high-quality ECG signals to be automatically identified and selected from the plurality of ECG signals obtained during cardiac monitoring to ensure any further processing is reliable.

The proposed invention determines the suitability of a given ECG signal by analysing activation patterns of a neural network trained to take a plurality of ECG signals as input and output a property of the plurality of ECG signals. Purely by way of example, the neural network model may employ a Convolutional Neural Network.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformations (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

There are several types of neural network, such as convolutional neural networks (CNNs) and recurrent neural networks (RNNs). CNNs typically contain several layers, including a convolutional layer, a pooling layer, and a fully connected layer. The convolutional layer consists of a set of learnable filters and extracts features from the input. The pooling layer is a form of non-linear down-sampling, reducing the data size by combining the outputs of a plurality of neurons in one layer into a single neuron in the next layer. The fully connected layer connects each neuron in one layer to all the neurons in the next layer.

Methods of training a neural network model are well known. Typically, such methods comprise obtaining a training dataset, comprising training input data entries and corresponding training output data entries. An initialized neural network algorithm is applied to each input data entry to generate predicted output data entries. An error between the predicted output data entries and corresponding training output data entries is used to modify the weights of the neural network algorithm. This process can be repeated until the error converges, and the predicted output data entries are sufficiently similar (e.g. $\pm 1\%$) to the training output data entries. This is commonly known as a supervised learning technique.

For example, weightings of the mathematical operation of each neuron may be modified until the error converges. Known methods of modifying a neural network include gradient descent, backpropagation algorithms and so on.

In a typical neural network, the mathematical operation applied at each node may be generalised to the form:

$$y = f(\textstyle\sum_i w_i x_i + b), \qquad\qquad (1)$$

where $x_i$ are the inputs to the node, $w_i$ are a set of input specific weights applied to the inputs $x_i$, $b$ is the bias of the node and $f$( ) is an activation function of the node. The weighting factors need not be one dimensional and therefore the output $y$ may not have the same dimensions as the inputs $x_i$. Common activation functions include ridge activation functions (such as linear activation or ReLU activation), radial activation functions (such as the Gaussian) or folding activation functions. The activation function introduces non-linearity into the neural network and thereby increases the complexity of the tasks the neural network can perform. In practice the activation function often involves thresholding, in which signals below a certain threshold level are set to have an output of zero and therefore do not get passed onto the next layer of the neural network. The activation level of the nodes of the neural network may therefore describe the result of applying the activation function at the node in the level of the output.

The output of a given node of the neural network model may often be referred to as a feature map. Therefore, each layer of the neural network is typically associated with a number of different feature maps corresponding to each node in the layer. Comparing the feature maps of each layer of the neural network to the input data allows us to gain some insight into which features of the input data are being used to generate the output of the neural network. Features of the input data that remain relatively unchanged in the feature maps contribute more to the decision of the neural network. Class activation maps (CAPs) may be generated from feature maps of the final layer of a neural network and describe the relative importance of each element of the input data for the determination of a given output class value generated by the neural network. The proposed invention leverages some of the features discussed above in the determination of the suitability of a plurality ECG signals for use in monitoring a cardiac system of a subject by determining an activation pattern for each ECG signal of a plurality of ECG signals.

The activation pattern associated with each ECG signal of the present invention describe the signal-specific activation level of the layers of a neural network when it is used to determine a property of the plurality of ECG signals. By this it is meant that the activation pattern associated with each ECG signal comprises information about the values of the outputs of the nodes of the neural network as they relate to the specific ECG signal the activation pattern is associated with. For example, the activation pattern of an ECG signal may trace the data of the ECG signal through the neural network and describe the activation level of the nodes that utilise this data. This information may be mapped back onto the ECG signal to describe the relative importance of each portion of the ECG signal for the determination of the property of the plurality of ECG signals.

By way of summary, the following steps outline a process according to the proposed concept(s):

Step 1: obtain a plurality of ECG signals intended to be used for cardiac monitoring of a subject. These are obtained directly from an ECG monitor or may be extracted from a database.
Step 2: process the plurality of ECG signals with a trained neural network model. The model may be capable of interpreting ECG waveforms and identifying salient characteristics of properties of the waveforms.
Step 3: determine an activation pattern for each ECG signal describing an associated activation level of the neural network when it is used to identify properties of the signals. This helps to identify which ECG signal is most important for determining features of the waveforms.
Step 4: determine a quality value for each ECG signal based on its activation pattern to describe the suitability of the signal for use in monitoring the cardiac system.

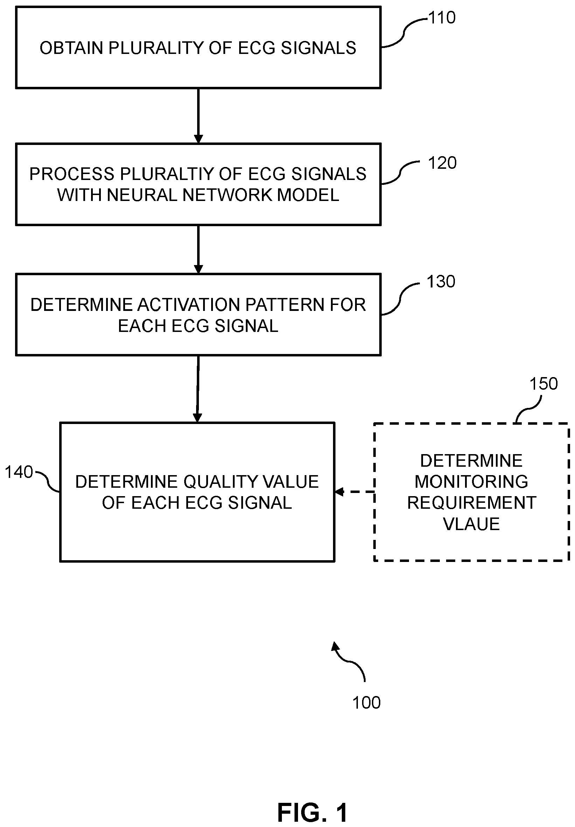

Referring now to Fig. 1, there is depicted a simplified flow diagram of a method 100 for determining the suitability of ECG signals for use in monitoring a cardiac system of a subject.

The method commences with a step 110 comprising obtaining a plurality of ECG signals for monitoring the cardiac system of the subject. In this embodiment, each of the plurality of ECG signals is obtained by a different lead of an ECG monitor.

The method then proceeds to step 120 comprising processing the plurality of ECG signals with a neural network model to determine a property of the plurality of ECG signals. In this exemplary method, the neural network model comprises a delineation model configured to determine at least one of: a location of heart beats within the plurality of ECG signals; a location of P waves within the plurality of ECG signals; a location of QRS waves within the plurality of ECG signals; a type of beat generating the QRS wave; a location of T waves within the plurality of ECG signals; an interval of a PR segment; an interval of a QT segment; and an interval of an ST segment.

Thus, the neural network model has been trained using annotated training data to identify salient global properties (characteristic values) of the plurality of ECG signals. In this case, training input data entries correspond to real or simulated ECG signals of a cardiac system, wherein each signal describes the electrical activity of the cardiac system over the same time period. The training output data entries correspond to properties of the plurality of ECG signals, such as those listed above, as manually determined by a health care professional. In other words, the neural network model used in step 120 of the method 100 has been trained using a deep learning algorithm configured to receive an array of training inputs and respective known outputs, wherein a training input comprises a plurality of ECG signals and a respective known output comprises a property of the plurality of ECG signals.

Once the property of the plurality of ECG signals has been determined, the method 100 proceeds to step 130 comprising, for each ECG signal of the plurality of ECG signals determining an activation pattern associated with the ECG signal, the activation pattern describing a signal-specific activation level of layers of the neural network model when the neural network model is used to determine the property of the plurality of ECG signals. Thus, the activation pattern associated with a given ECG signal comprises information about the output value of the nodes of the neural network model and the proportion of this output that is derived from the associated ECG signal. The activation pattern of the neural network associated with each ECG signal may therefore act as an indicator of the level of importance the particular ECG signal had in the determination of the property of the plurality of ECG signals. Noisier signals will cause lower activation levels of the neural network as they are less useful for determining the property of the cardiac signal. The activation pattern associated with a given ECG signal may therefore act as a metric for the quality of the ECG signal.

Step 140 comprises determining for each ECG signal of the plurality of ECG signals, based on the determined activation patterns associated with the ECG signal, a quality value of the ECG signal indicating the suitability of the ECG signal for monitoring the cardiac system of the subject. The quality value may comprise any suitable quantitative or qualitative score of the ECG signal that indicates a predicted usefulness of the ECG signal for monitoring the cardiac system.

In this exemplary embodiment the method further comprises step 150 comprising determining a monitoring requirement value, describing a configuration of ECG signals required for monitoring the cardiac system of the subject. This information may be incorporated into the determination of the quality value of each of the ECG signals. For example, the monitoring requirement value may comprise at least one of: a number of ECG signals required for monitoring the cardiac system of the subject; and a type of ECG signal required for monitoring the cardiac system of the subject. Different aspects of cardiac monitoring may have different requirements on the number and type of signals that are required. For example, in real-time monitoring applications for patient surveillance, a lead limb (such as I, II, or III) and a chest lead (V1, V2, V3, etc.) may be preferred to identify onset of critical arrhythmias. The monitoring requirement value may also contain information about other monitoring specific factors such as the level of noise in the ECG signal that is acceptable. In this exemplary embodiment, information about such ECG signal configurations may be used in the determination of the quality value of each ECG signal such that it more accurately reflects the suitability of the signals for the type of cardiac monitoring that is to be carried out.

Alternative embodiments of the present invention may not include the step 150. Thus, a generic quality value may be determined describing a general suitability of the ECG signal for use in monitoring the patient which is not specific to any interpretation algorithm to be applied to the signal. This may be beneficial in allowing the flexibility for the quality value to be used by a wide variety of different systems and methods to determine whether or not to use the ECG signal in further processing steps.

Similarly, other aspects of the present invention may be executed differently in alternative embodiments. Although discussed mainly in the context of ECG lead selection, the concepts of the present invention may be equally well applied to ECG signals obtained from separate monitoring systems rather than different leads of the same monitor. When used for lead selection, each of the ECG signals may relate to the same time period of cardiac monitoring and therefore depict the same period of electrical activity of the subject's heart. However, in other embodiments each of the plurality of ECG signals may relate to different time periods (which may or may not overlap) so long as the signals share at least one common property (e.g. resting heart rate, given time interval within a heartbeat).

The quality score determined in the proposed invention may take many forms. In some embodiments, the quality score of an ECG signal may comprise a rank of the ECG signal describing a relative importance of the ECG signal compared to the others of the plurality of ECG signals for the determination of the property of the plurality of ECG signals. In other embodiments, the quality score may directly indicate the usefulness of the signal for use in certain diagnosis or patient assessment and surveillance properties. In particular the quality value may describe the suitability of the ECG signal for detecting arrhythmia of the cardiac system of the subject (the detection of heart arrythmia may be particularly sensitive to noise in the ECG signal).

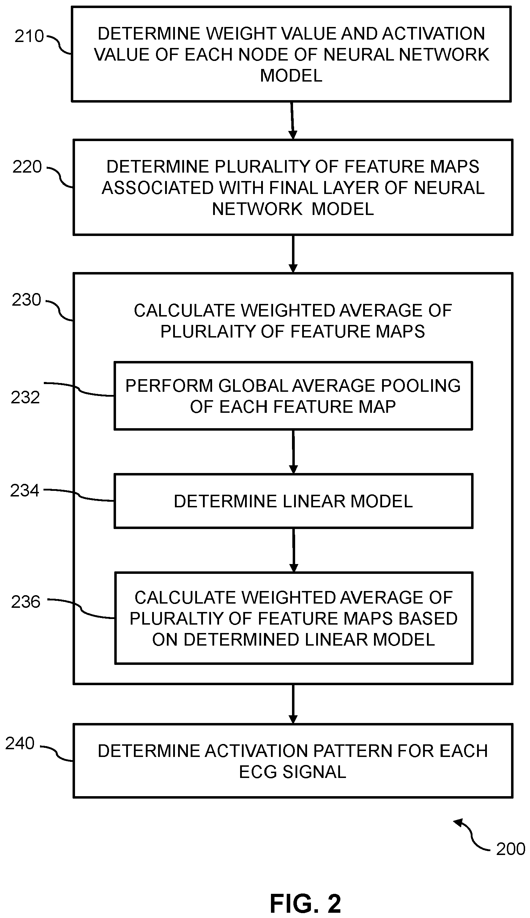

Referring now to Fig. 2, there is depicted a simplified flow diagram of a method 200 for determining an activation pattern associated with an ECG signal according to a proposed embodiment. In certain proposed embodiments this method may be used within step 130 of the method 100.

The method commences with a step 210 comprising determining a weight value and an activation value associated with each of a plurality of nodes of the neural network when the neural network is used to determine the characteristic of the plurality of ECG signals. These correspond to the values of $w_i$ and $y$ in Equation 1 outlined above.

Step 220 comprises determining a plurality of feature maps associated with the final layer of the neural network model. For convolutional neural networks, this may comprise the final convolutional layer of the network. As discussed above, the feature maps correspond to the output of each node in the layer.

In step 230, a weighted average of the plurality of feature maps associated with the final layer of the neural network model is determined. To calculate the weighting factors for this weighted average, sub-steps 232, 234, and 236 are carried out.

Step 232 comprises processing each of the plurality of feature maps with a global average pooling function to determine a global average value of the feature map. This involves taking an arithmetic mean value of all the data values in the feature map.

Step 234 comprises determining a linear model, based on the determined global average values of the plurality of feature maps, which maps the global average values of the plurality of feature maps to the determined property (or properties) of the plurality of ECG signals as output by the neural network model. This linear model may in some embodiments be learned using an additional neural network model. The linear model comprises a weighting factor for each of the global average values such that when applied and added together, the predetermined value of the property of the plurality of ECG signals is obtained.

Step 236 comprises calculating a weighted average of the plurality of feature maps using the weighting factors of the linear model. In other words, the weighting factor applied to the global average value of a given feature map is applied to the feature map itself in the calculation of the weighted average of the plurality of feature maps.

Once this weighted average of the plurality of feature maps has been determined, the method proceeds to step 240 comprising determining, for each ECG signal of the plurality of ECG signals, based on the weighted average of the plurality of feature maps, an activation pattern associated with the ECG signal that describes the activation level of layers of the neural network model when the neural network model is used to determine the property of the plurality of ECG signals. This involves mapping the weighted average of feature maps onto the plurality of ECG signals to determine the relative importance of each portion of each of the plurality of ECG signals in the determination of the property of the plurality of ECG signals.

Figure 2 depicts one way in which signal-specific activation patterns may be determined, however other embodiments may utilise different methods. For example, the activation level of each node of the neural network may be used directly, along with information of the weighting of each input to the node to calculate an overall activation level within the neural network for each portion of each of the plurality of ECG signals, without requiring global average pooling of feature maps.

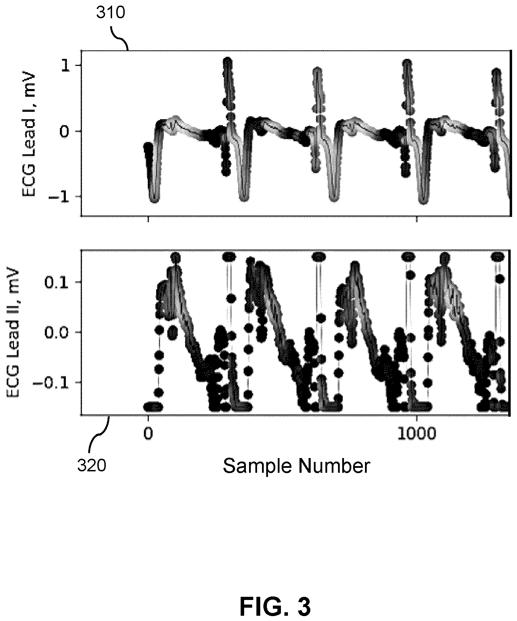

Referring now to Fig. 3, there is depicted example activation patterns for two different ECG signals.

The pattern depicted in the upper signal plot/representation of Figure 3, depicts a first activation pattern 310 of a first ECG signal. The first ECG signal describes the measured electrical activity of a cardiac system from a specific lead of an ECG monitor in mV. Each portion of the ECG signal has been shaded with a grayscale value indicating the activation level of the layers of the neural network that is associated with that portion of the ECG signal. Lighter shading indicates portions for which the activity level is high, and darker shading indicates portions for which the activity level is low.

The pattern depicted in the lower plot/representation of Figure 3, depicts a second activation pattern 320 of a second ECG signal. The second ECG signal is obtained by a second lead of the ECG monitor and corresponds to the same time period of cardiac monitoring as the first ECG signal. The second ECG signal has been shaded in grayscale according to the activation level associated with each portion of the second ECG signal in the same way as the first signal.

By eye, it is easy to establish that the second signal has suffered more extensive noise than the first signal. This fact is also reflected in the activation patterns of the two signals. The activation levels associated with the first signal are generally higher than the activation levels associated with the second ECG signal and further the portions associated with low activation are much less extensive.

Fig. 3 shows how the activation pattern associated with an ECG signal may indicate a level of noise present in the signal and therefore may be a useful metric for the quality of the signal and hence how useful it may be for monitoring the cardiac system.

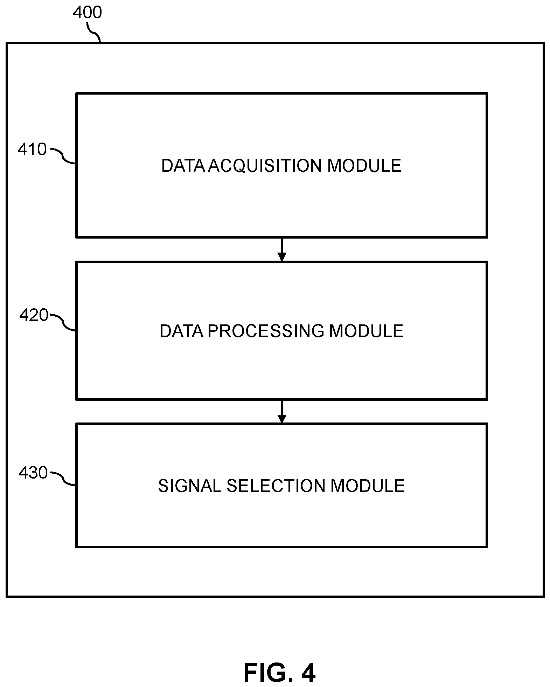

Referring now to Fig. 4, there is depicted a simplified block diagram of a system 400 for determining the suitability of ECG signals for monitoring a cardiac system of a patient.

The system 400 comprises a data acquisition module 410, a data processing module 420, and a signal selection module 430. The system 420 is configured to carry out the method 100 described above.

In detail the data acquisition module 410 is configured to obtain a plurality of ECG signals for monitoring the cardiac system of the subject. In some embodiments, the data acquisition module may itself comprise an echocardiogram monitor, in this way the proposed system may be integrated into a patient monitor. In other embodiments, the system is configured to exist as a separate device to an echocardiogram monitor and the data acquisition module is configured to communicate with such a monitor to obtain the plurality of ECG signals.

The data processing module 420 is configured to: process the plurality of ECG signals with a neural network model to determine a property of the plurality of ECG signals; and for each ECG signal of the plurality of ECG signals determine an activation pattern associated with the ECG signal describing a signal-specific activation level of layers of the neural network model when the neural network model is used to determine the characteristic of the plurality of ECG lead signals. The determined activation patterns are then passed to the signal selection module 430.

The signal selection module 430 is configured to, for each ECG signal of the plurality of ECG signals, determine, based on the determined activation maps of the plurality of ECG signals, a quality value of the ECG signal indicating the suitability of the ECG signal for monitoring the cardiac system of the subject. In some embodiments this may involve selecting the subset of the plurality of ECG signals for further processing.

The system 400 may be integrated into existing ECG monitors or may exist as a standalone device. Further functionalities such as further automatic monitoring algorithms including arrhythmia detection and ECG segmentation may be carried out based on the quality scores calculated by the system 400. For example, the system 400 may be configured to automatically select an optimal subset of the plurality of ECG signals for use in monitoring the cardiac system of the subject and then use a rule-based or feature-based algorithm to automatically analyse the cardiac system based on the selected subset of the plurality of ECG signals.

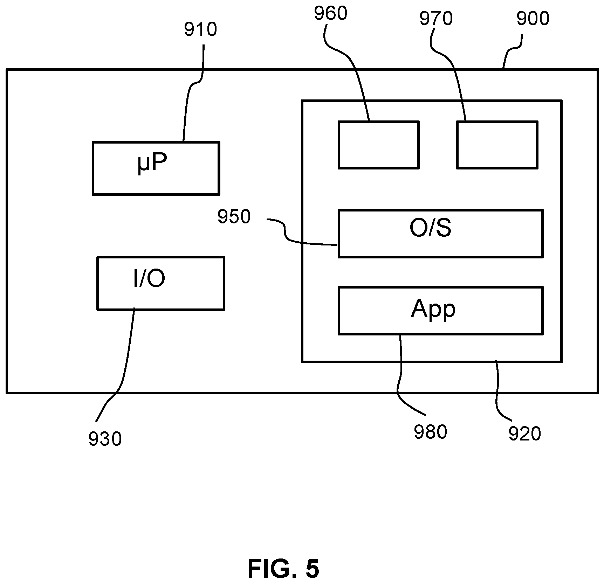

Fig. 5 illustrates an example of a computer 900 within which one or more parts of an embodiment may be employed. Various operations discussed above may utilize the capabilities of the computer. For example, one or more parts of a proposed embodiment may be incorporated in any element, module, application, and/or component discussed herein. In this regard, it is to be understood that system functional blocks can run on a single computer or may be distributed over several computers and locations (e.g. connected via internet), such as a cloud-based computing infrastructure.

The computer 900 includes, but is not limited to, PCs, workstations, laptops, PDAs, palm devices, servers, storages, and the like. Generally, in terms of hardware architecture, the computer 900 may include one or more processors 910, memory 920 and one or more I/O devices 930 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 910 is a hardware device for executing software that can be stored in the memory 920. The processor 910 can be virtually any custom made or commercially available processor, a central processing unit (CPU), a digital signal processor (DSP), or an auxiliary processor among several processors associated with the computer 900, and the processor 910 may be a semiconductor based microprocessor (in the form of a microchip) or a microprocessor.

The memory 920 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 920 may incorporate electronic, magnetic, optical, and/or other types of storage media. Note that the memory 920 can have a distributed architecture, where various components are situated remote from one another, but can be accessed by the processor 910.

The software in the memory 920 may include one or more separate programs, each of which comprises an ordered listing of executable instructions for implementing logical functions. The software in the memory 920 includes a suitable operating system (O/S) 950, compiler 960, source code 970, and one or more applications 980 in accordance with exemplary embodiments. As illustrated, the application 980 comprises numerous functional components for implementing the features and operations of the exemplary embodiments. The application 980 of the computer 900 may represent various applications, computational units, logic, functional units, processes, operations, virtual entities, and/or modules in accordance with exemplary embodiments, but the application 980 is not meant to be a limitation. The operating system 950 controls the execution of other computer programs, and provides scheduling, input-output control, file and data management, memory management, and communication control and related services. It is contemplated by the inventors that the application 980 for implementing exemplary embodiments may be applicable on all commercially available operating systems.

Application 980 may be a source program, executable program (object code), script, or any other entity comprising a set of instructions to be performed. When a source program, then the program is usually translated via a compiler (such as the compiler 960), assembler, interpreter, or the like, which may or may not be included within the memory 920, so as to operate properly in connection with the O/S 950. Furthermore, the application 980 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like. The I/O devices 930 may include input devices such as, for example but not limited to, a mouse, keyboard, scanner, microphone, camera, etc. Furthermore, the I/O devices 930 may also include output devices, for example but not limited to a printer, display, etc. Finally, the I/O devices 930 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 900 is a PC, workstation, intelligent device or the like, the software in the memory 920 may further include a basic input output system (BIOS) (omitted for simplicity). The BIOS is a set of essential software routines that initialize and test hardware at start-up, start the O/S 950, and support the transfer of data among the hardware devices. The BIOS is stored in some type of read-only-memory, such as ROM, PROM, EPROM, EEPROM or the like, so that the BIOS can be executed when the computer 900 is activated. When the computer 900 is in operation, the processor 910 is configured to execute software stored within the memory 920, to communicate data to and from the memory 920, and to generally control operations of the computer 900 pursuant to the software. The application 980 and the O/S 950 are read, in whole or in part, by the processor 910, perhaps buffered within the processor 910, and then executed.

When the application 980 is implemented in software it should be noted that the application 980 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed method(s), device(s) and/or system(s) may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer-readable storage medium stores a computer program comprising computer program code configured to cause one or more physical computing devices to carry out a control method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, some of the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. In this regard, each block in the flow diagrams or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flow diagrams and combinations of blocks in the block diagrams and/or flow diagrams, can be implemented by special purpose hardware-based systems that perform the specified functions or acts or carry out combinations of special purpose hardware and computer instructions.

**Claims**

1. A computer-implemented method (100) for determining the suitability of electrocardiogram (ECG) signals for monitoring a cardiac system of a subject, the method comprising:

   obtaining (110) a plurality of ECG signals for monitoring the cardiac system of the subject;
   processing (120) the plurality of ECG signals with a neural network model to determine a property of the plurality of ECG signals;
   for each ECG signal of the plurality of ECG signals determining (130) an activation pattern associated with the ECG signal, the activation pattern describing a signal-specific activation level of layers of the neural network model when the neural network model is used to determine the property of the plurality of ECG signals; and

for each ECG signal of the plurality of ECG signals determining (140) a quality value of the ECG signal, based on the determined activation patterns, indicating the suitability of the ECG signal for monitoring the cardiac system of the subject.

2. The method of claim 1, wherein the method further comprises determining (150) a monitoring requirement value, describing a configuration of ECG signals required for monitoring the cardiac system of the subject, and wherein determining for each ECG signal of the plurality of ECG signals a suitability of the ECG signal for monitoring the cardiac system of the subject is further based on the determined monitoring requirement value.

3. The method of claim 2, wherein the monitoring requirement value comprises at least one of:
a number of ECG signals required for monitoring the cardiac system of the subject; and
a type of ECG signal required for monitoring the cardiac system of the subject.

4. The method of any preceding claim, wherein determining the activation pattern associated with an ECG signal of the plurality of ECG signals comprises:
Determining (210) a weight value and an activation value associated with each of a plurality of nodes of the neural network when it is used to determine the characteristic of the plurality of ECG signals; and
determining (240) the activation pattern for the ECG signal based on the determined weight and activation values of the plurality of nodes of the neural network.

5. The method of any preceding claim, wherein determining the activation pattern associated with an ECG signal of the plurality of ECG signals comprises:

   determining (220) a plurality of feature maps associated with a final layer of the neural network model,
   calculating (230) a weighted average of the plurality of feature maps; and
   determining (240) the activation pattern for the ECG signal of the plurality of ECG signals based on a weighted average of the plurality of feature maps.

6. The method of any preceding claim, wherein the property of the plurality of ECG signals comprises at least one of:

   a location of heart beats within the plurality of ECG signals;
   a location of P waves within the plurality of ECG signals;
   a location of QRS waves within the plurality of ECG signals;
   a type of beat generating the QRS wave;
   a location of T waves within the plurality of ECG signals;
   an interval of a PR segment;
   an interval of a QT segment; and
   an interval of an ST segment.

7. The method of any preceding claim, wherein each of the plurality of ECG signals is obtained by a different lead of an ECG monitor.

8. The method of any preceding claim, wherein the quality value of an ECG signal of the plurality of ECG signals comprises a rank of the ECG signal describing a relative importance of the ECG signal compared to the other of the plurality of ECG signals for the determination of the characteristic of the plurality of the ECG signals.

9. The method of any preceding claim, wherein the quality value of an ECG signal of the plurality of ECG signals describes the suitability of the ECG signal for monitoring the cardiac system of the subject during a predetermined time period.

10. The method of any preceding claim, wherein the quality value of an ECG signal of the plurality of ECG signals describes the suitability of the ECG signal for detecting arrhythmia of the cardiac system of the subject.

11. A computer program comprising computer program code means adapted, when said computer program is run on a computer, to implement the method of any of claims 1-10.

12. A system (400) for determining the suitability of electrocardiogram (ECG) signals for use in monitoring a cardiac system of a subject, the system comprising:

a data acquisition module (410) configured to obtain a plurality of ECG signals for monitoring the cardiac system of the subject; and
a data processing module (420) configured to:

process the plurality of ECG signals with a neural network model to determine a property of the plurality of ECG signals; and
for each ECG signal of the plurality of ECG signals determine an activation map of the ECG signal describing an activation level of layers of the neural network model when the neural network model is used to determine the characteristic of the plurality of ECG lead signals; and
a signal selection module (430) configured to, for each ECG signal of the plurality of ECG signals, determine a quality value of the ECG signal, based on the determined activation maps of the plurality of ECG signals, indicating the suitability of the ECG signal for monitoring the cardiac system of the subject.

13. The system of claim 12 further comprising an ECG monitor configured to measure the plurality of ECG signals for monitoring the cardiac system of the subject.

14. The system of any of claims 12 and 13, wherein the data acquisition module is further configured to determine a monitoring requirement value, describing a configuration of ECG signals required for monitoring the cardiac system of the subject, and
wherein the signal selection module is further configured to determine for each ECG signal of the plurality of ECG signals, a suitability of the ECG signal for monitoring the cardiac system of the subject based on the determined monitoring requirement value and the determined activation patterns.

15. The system of claim 14, wherein the monitoring requirement comprises at least one of: a number of ECG signals required for monitoring the cardiac system of the subject; and
a type of ECG signal required for monitoring the cardiac system of the subject.

OBTAIN PLURALITY OF ECG SIGNALS — 110

PROCESS PLURALTIY OF ECG SIGNALS WITH NEURAL NETWORK MODEL — 120

DETERMINE ACTIVATION PATTERN FOR EACH ECG SIGNAL — 130

150

140 — DETERMINE QUALITY VALUE OF EACH ECG SIGNAL

DETERMINE MONITORING REQUIREMENT VLAUE

100

**FIG. 1**

210 — DETERMINE WEIGHT VALUE AND ACTIVATION VALUE OF EACH NODE OF NEURAL NETWORK MODEL

220 — DETERMINE PLURALITY OF FEATURE MAPS ASSOCIATED WITH FINAL LAYER OF NEURAL NETWORK MODEL

230 — CALCULATE WEIGHTED AVERAGE OF PLURLAITY OF FEATURE MAPS

232 — PERFORM GLOBAL AVERAGE POOLING OF EACH FEATURE MAP

234 — DETERMINE LINEAR MODEL

236 — CALCULATE WEIGHTED AVERAGE OF PLURALTIY OF FEATURE MAPS BASED ON DETERMINED LINEAR MODEL

240 — DETERMINE ACTIVATION PATTERN FOR EACH ECG SIGNAL

200

**FIG. 2**

FIG. 3

400

410 — | DATA ACQUISITION MODULE |

420 — | DATA PROCESSING MODULE |

430 — | SIGNAL SELECTION MODULE |

**FIG. 4**

**FIG. 5**

## EP 4 670 633 A1

Europäisches
Patentamt
European
Patent Office
Office européen
des brevets

**EUROPEAN SEARCH REPORT**

**Application Number**

EP 24 18 4436

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MONDAL ACHINTA ET AL: "Automatic ECG Signal Quality Determination Using CNN With Optimal Hyperparameters for Quality-Aware Deep ECG Analysis Systems", IEEE SENSORS JOURNAL, IEEE, USA, [Online] vol. 24, no. 11, 3 April 2024 (2024-04-03), pages 17825-17833, XP011971570, ISSN: 1530-437X, DOI: 10.1109/JSEN.2024.3382720 Retrieved from the Internet: URL:https://ieeexplore.ieee.org/stamp/stamp.jsp?arnumber=10491116> [retrieved on 2024-04-04] * abstract * * sect.I.B.1;II.C;III.A;IV * * figures 1,4 * * figure in abstract * | 1-15 | INV. A61B5/349 A61B5/00 A61B5/366 G06N3/044 G06N3/0464 G16H50/20 |
| X | RUFAI AHMAD ET AL: "Using Artificial Neural Networks to Diagnose Heart Disease", INTERNATIONAL JOURNAL OF COMPUTER APPLICATIONS : IJCA, [Online] vol. 182, no. 19, 19 October 2018 (2018-10-19), pages 1-6, XP093231553, US ISSN: 0975-8887, DOI: 10.5120/ijca2018917938 Retrieved from the Internet: URL:https://www.ijcaonline.org/archives/volume182/number19/rufai-2018-ijca-917938.pdf> [retrieved on 2024-12-06] * abstract * * sect.2;4 * | 1-15 | TECHNICAL FIELDS SEARCHED (IPC) A61B G06N G16H |

-/--

The present search report has been drawn up for all claims

| Place of search | Date of completion of the search | Examiner |
|---|---|---|
| The Hague | 6 December 2024 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 4

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MITRA MALAY ET AL: "Cardiac Arrhythmia Classification Using Neural Networks with Selected Features", PROCEDIA TECHNOLOGY, [Online] vol. 10, 1 January 2013 (2013-01-01), pages 76-84, XP093231560, NL ISSN: 2212-0173, DOI: 10.1016/j.protcy.2013.12.339 Retrieved from the Internet: URL:https://www.sciencedirect.com/science/article/pii/S2212017313004933/pdf?md5=8a4b035f0245c74268154cea5536ca6c&pid=1-s2.0-S2212017313004933-main.pdf> [retrieved on 2024-12-06] * abstract * * sect.3;4;5.3 * * figure 2 * | 1-15 | |
| X | US 2021/100471 A1 (YU LONG [US] ET AL) 8 April 2021 (2021-04-08) * abstract * * paragraphs [0054] - [0063], [0088], [0096], [0128], [0133], [0138]; claims 9-16; figure 12 * | 1-15 | TECHNICAL FIELDS SEARCHED (IPC) |

-----

-/--

The present search report has been drawn up for all claims

| Place of search | Date of completion of the search | Examiner |
|---|---|---|
| The Hague | 6 December 2024 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 670 633 A1**

Europäisches
Patentamt

European
Patent Office

Office européen
des brevets

## EUROPEAN SEARCH REPORT

**Application Number**

EP 24 18 4436

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MONDAL ACHINTA ET AL: "Fast CNN-Based Electrocardiogram Signal Quality Assessment Using Fourier Magnitude Spectrum for Resource-Constrained ECG Diagnosis Devices", IEEE SENSORS LETTERS, [Online] vol. 8, no. 4, 1 April 2024 (2024-04-01), pages 1-4, XP093231593, ISSN: 2475-1472, DOI: 10.1109/LSENS.2024.3371479 Retrieved from the Internet: URL:https://ieeexplore.ieee.org/stampPDF/g etPDF.jsp?tp=&arnumber=10453615&ref=aHR0cH M6Ly9pZWVleHBsb3JlLmllZWUub3JnL2RvY3VtZW50 LzEwNDUzNjE1> [retrieved on 2024-12-06] * abstract * * sect.II, III * ----- | 1-4,8-15 | |
| X | JIE SUN: "Automatic cardiac arrhythmias classification using CNN and attention-based RNN network", HEALTHCARE TECHNOLOGY LETTERS, JOHN WILEY & SONS, INC, HOBOKEN, USA, [Online] vol. 10, no. 3, 20 April 2023 (2023-04-20) , pages 53-61, XP006118569, ISSN: 2053-3713, DOI: 10.1049/HTL2.12045 Retrieved from the Internet: URL:https://ietresearch.onlinelibrary.wile y.com/doi/epdf/10.1049/htl2.12045> [retrieved on 2024-12-06] * abstract * * sect.3 * * figures 1,2 * ----- -/-- | 1-6,8-15 | **TECHNICAL FIELDS SEARCHED (IPC)** |

The present search report has been drawn up for all claims

| Place of search | Date of completion of the search | Examiner |
|---|---|---|
| The Hague | 6 December 2024 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 4

Europäisches
Patentamt

European
Patent Office

Office européen
des brevets

**EUROPEAN SEARCH REPORT**

Application Number

EP 24 18 4436

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/020293 A1 (RICHARDS DYLAN [US]) 21 January 2021 (2021-01-21) * abstract * * paragraph [0175]; claim 19; figure 7 * ----- | 1-15 | |
| A | PEDRYCZ ET AL: "The design of cognitive maps: A study in synergy of granular computing and evolutionary optimization", EXPERT SYSTEMS WITH APPLICATIONS, ELSEVIER, AMSTERDAM, NL, [Online] vol. 37, no. 10, 1 October 2010 (2010-10-01), pages 7288-7294, XP027067525, ISSN: 0957-4174, DOI: 10.1016/J.ESWA.2010.03.006 Retrieved from the Internet: URL:https://www.sciencedirect.com/science/article/pii/S0957417410001879/pdfft?md5=1698f10de6ecd6895550ab474fc40b29&pid=1-s2.0-S0957417410001879-main.pdf> [retrieved on 2024-12-05] * abstract * * the whole document * * sect.2-4 * ----- | 1-15 | |

TECHNICAL FIELDS SEARCHED (IPC)

The present search report has been drawn up for all claims

| Place of search | Date of completion of the search | Examiner |
|---|---|---|
| The Hague | 6 December 2024 | Delval, Christophe |

page 4 of 4

## ANNEX TO THE EUROPEAN SEARCH REPORT
## ON EUROPEAN PATENT APPLICATION NO.

EP 24 18 4436

This annex lists the patent family members relating to the patent documents cited in the above-mentioned European search report.
The members are as contained in the European Patent Office EDP file on
The European Patent Office is in no way liable for these particulars which are merely given for the purpose of information.

06-12-2024

| Patent document cited in search report | Publication date | Patent family member(s) | | Publication date |
|---|---|---|---|---|
| US 2021100471 A1 | 08-04-2021 | CN | 114901143 A | 12-08-2022 |
| | | EP | 4042445 A1 | 17-08-2022 |
| | | US | 2021100471 A1 | 08-04-2021 |
| | | US | 2023143594 A1 | 11-05-2023 |
| | | WO | 2021071688 A1 | 15-04-2021 |
| US 2021020293 A1 | 21-01-2021 | CN | 114616628 A | 10-06-2022 |
| | | EP | 4000074 A1 | 25-05-2022 |
| | | US | 2021020293 A1 | 21-01-2021 |
| | | US | 2023033967 A1 | 02-02-2023 |
| | | US | 2024347164 A1 | 17-10-2024 |
| | | WO | 2021011381 A1 | 21-01-2021 |

EPO FORM P0459

For more details about this annex : see Official Journal of the European Patent Office, No. 12/82